# EUROPEAN PATENT SPECIFICATION

(11) **EP 0 864 864 B1**
(45) Date of publication and mention of the grant of the patent: **02.01.2003**
(21) Application number: 98301783.1
(22) Date of filing: 11.03.1998
(51) Int. Cl.: G01N 33/52, G01N 33/53, G01N 33/573, G01N 33/68, C12Q 1/00, C12Q 1/37, C12Q 1/28, C12Q 1/32, C12Q 1/42

(54) **Method and apparatus for mapping the condition of a wound**
Methode und Gerät zum Aufzeichnen des Zustandes einer Wunde
Méthode et appareil de traçage d'état d'une blessure

(30) Priority: 12.03.1997 GB 9705081
(43) Date of publication of application: 16.09.1998
(73) Proprietor: Johnson & Johnson Medical Ltd., Edinburgh EH2 4NH (GB)
(72) Inventor: Watt, Paul William, West Yorkshire BD20 6UN (GB); Harvey, Wilson, Gargunnock, Stirling FK8 3AX (GB); Grady, Michael, Menston, West Yorkshire LS23 6BY (GB); McCabe, John Patrick, Skipton BD23 1HL (GB); Tarlton, John, Horfield, Bristol BS7 8QY (GB)
(74) Representative: Fisher, Adrian John

(56) References cited:
- DE-A- 3 715 245
- GÖKHAN, A et al. "Extracellular Alkaline Phosphatase Activity as a Possible Marker for Wound Healing: A preliminary Report" Journal of Oral and Maxillofacial Surgery, January 1997, Vol. 55, No. 1, pages 56-62, XP002900144

## Description

The present invention relates to reactive sheet materials for mapping the condition of a wound, and methods for carrying out such diagnostic mapping.

It is known that large area wounds can show different wound pathologies and different healing rates at different locations on the wound surface. This applies especially to large-area bums, and to large-area chronic wounds such as diabetic ulcers, decubitis ulcers and venous ulcers. Optimization of wound diagnosis and treatment may be assisted by having a map showing the condition of different regions of the wound.

DE-A-37 15 245 describes a process for the production of a dry type analytical element for determining alkaline phosphatase. The analytical element includes a reactive layer comprising p-nitrophenyl phosphate which acts as a substrate for the alkaline phosphatase.

Certain existing methods of determining the condition of a wound rely on analyzing wound fluid that has been removed from the wound surface by a swab or the like. Clearly, the composition of such wound fluid is averaged over the whole surface of the wound, and is not normally representative of any specific area of the wound.

Accordingly, it is an object of the present invention to provide methods for mapping the condition of a wound, in particular for mapping the presence of biochemical wound markers. It is a further object of the present invention to provide an apparatus specifically adapted for use in such a method.

The present invention provides a diagnostic sheet for mapping the condition of a wound, by application directly to the surface of a wound, wherein said sheet comprises a reactive layer that is selectively reactive over at least a part of its area with one or more molecules present in a wound fluid and a porous wound contacting layer.

The wound fluid can be transferred directly or indirectly (e.g. by blotting) from the wound surface onto the diagnostic sheet according to the present invention, whereupon the reaction between the molecules present in the wound fluid and the reactive part of the sheet can be used to provide a map of the concentration of the said molecules as a function of position in the wound.

The diagnostic sheet according to the present invention is especially suitable for use with large surface area wounds, for which purpose the reactive area of the diagnostic sheet is preferably greater than about 10cm², and more preferably greater than about 16cm². Preferably, the reactive area of the sheet is substantially round or square. Preferably, the whole of the diagnostic sheet is uniformly reactive with the said molecules present in the wound fluid.

Preferably, the diagnostic sheet contains one or more immunological binding partners to bind the one or more molecules present in the wound fluid. The immunological binding partners may for example comprise monoclonal or polyclonal antibodies, antibody fragments, or chimeric antibodies. Alternatively, the immunological binding partners may comprise antigens in cases where the presence of predetermined antibodies in the wound fluid is being mapped. Preferably, the immunological binding partners comprise monoclonal antibodies. Preferably, the immunological or other binding partners are immobilised on the sheet, for example by avidin-biotin linking, or dialdehyde derivatization of the sheet material followed by cross-linking to a peptide binding partner.

The sheets of material according to the present invention comprising immunological or other binding partners may be used in a range of immunoassays to map the presence of biologically active molecules in large-area wounds. For example, the sheets having antibodies or antibody fragments bound thereto may be used in sandwich immunoassay-type mapping. Alternatively, the sheet may have analog ligands bound to the antibodies, whereby the molecules present in the wound fluid are mapped by affinity displacement immunoassay. Various other immunoassays will be apparent to persons skilled in the art.

In other preferred embodiments, the diagnostic sheet according to the present invention comprises a chemiluminescent, chromogenic or fluorogenic substrate for an enzyme present in the wound fluid.

The sheet of material according to the present invention preferably comprises a plurality of layers. Preferably, these include an absorbent layer for absorbing the wound fluid. The absorbent layer may be the same as, or distinct from a reactive layer for reacting with the one or more molecules present in the wound fluid to map those molecules over the area of the wound. The reactive layer may be located on either side of the absorbent layer (i.e. facing the wound in use, or facing away from the wound in use), provided that wound fluid can pass into or through the reactive layer. The use of an absorbent layer facilitates uptake of the wound fluid and minimizes lateral flow of the wound fluid that could distort the wound map.

In its simplest aspect, the diagnostic sheet according to the present invention is applied to the surface of the wound, for direct absorption of wound fluid over the area of the sheet. For use in such methods the sheet material is preferably conformable, sterile, non-adherent and wound-friendly. Preferably, the sheet further comprises a porous wound contacting layer that permits the flow of wound fluid into the sheet of material, but prevents contamination of the sheet by wound debris. In certain preferred embodiments the wound contacting layer is microporous, with a pore size preferably in the range of 0.01 to 1µm. The use of a microporous film prevents bacterial or particulate contamination of the sheet of material, and may also be useful for some preliminary molecular weight selection of molecules present in the wound fluid. Suitable microporous films include microporous polyurethane films and microporous polyvinylidine fluoride (PVDF) films available from Pall Corporation.

In accordance with a further aspect, the invention provides a diagnostic sheet as above in a sterile package, such as a gamma-irradiated laminated foil pouch.

Preferably, the absorbent/reactive layers of the sheet of material according to the present invention comprise at least one layer of material selected from the group consisting of: gelatin, cellulose, nitrocellulose, agar, polyacrylamide, starch, alginate, bacterial or plant gums such as guar gum, xantham gum or locust bean gum, glass fiber, polyethylene, polycarbonate or polypropylene.

The sheets of material according to the present invention can be used to map substantially any detectable molecule that is present in wound fluid. The sheets may also be impregnated with an acid/base indicator to map the pH of the wound. Preferably, the sheet is reactive with one or more molecules that are indicative of a wound healing disorder. Preferably, the sheet is reactive with one or more molecules selected from the group consisting of: protease enzymes, collagen propeptides, collagen telopeptides and collagen crosslinks such as pyridinoline, protease inhibitors, plasmin, lactate dehydrogenase, cathepsins, cytokines, peroxidase enzymes, cortisol free radicals and growth factors. More preferably, the one or more molecules are protease enzymes selected from the group consisting of matrix metalloproteinases, low molecular weight gelatinases and latent or active elastases. Most preferably, the one or more molecules comprise matrix metalloproteinase 2 (MMP2) or matrix metalloproteinase 9 (MMP9). The protease enzymes are preferably detected by reaction with chromogenic or fluorogenic substrates bound to the sheet of material, such as Dnp-Pro-β-cyclohexyl-Ala-Gly-Cys(Mu)-His-Ala-Lys(N-Me-Abz)-NH₂ (available from Bachem Inc. - for MMP9 and 1). Other chromogenic substrates such as Gelatin-Texas Red and Gelatin-Azobrilliant Blue could be used to map these molecules.

In other preferred embodiments, the diagnostic sheet reacts with an alkaline phosphatase (ALP) enzyme present in the wound. It is known from F. Alpaslan, T. Nakajima and Y. Takano J. Oral. Maxillofac. Surg. Vol. 55, pp 56-62 (1997) that extracellular ALP levels are elevated in healing wounds. The diagnostic sheet could, for example, comprise a substrate for ALP such as p-nitrophenyl phosphate (see G. N. Price, Clin.Chim. Acta vol. 94, p.211, (1979). Alternatively or additionally, the diagnostic sheet could comprise an immunological binding partner for ALP.

A visible map can also be produced on the diagnostic sheet by various methods ) known in the art for immunoassay, such as sandwich immunoassay.

In a second aspect, the present invention provides a kit for mapping the condition of a wound, wherein the kit comprises a diagnostic sheet according to the present invention and an immunological binding partner for the one or more molecules, wherein the immunological binding partner is bound to an indicator molecule. Preferably, this is a kit for sandwich immunoassay. The indicator molecule may be any of the indicator molecules conventionally used in the art, such as horseradish peroxidase.

In a third aspect, the present invention provides the use of a diagnostic sheet according to the present invention for the preparation of a diagnostic wound dressing for application to the surface of a wound to map the condition of the wound.

In a fourth aspect, the present invention provides a method of mapping the condition of a wound comprising the steps of: applying an absorbent sheet to the surface of the wound to blot wound fluid from the wound; followed by applying the absorbent sheet to a diagnostic sheet according to the present invention to transfer a part of the wound fluid to the diagnostic sheet for said diagnostic mapping. Preferably, the diagnostic sheet is remote from the wound when the wound fluid is transferred to the diagnostic sheet.

Specific embodiments of the present invention will now be described further, by way of example.

### Example 1

An absorbent diagnostic wound mapping sheet in accordance with the present invention is prepared as follows. A sheet of Whatman (RTM) No. 1 filter paper (5 cm by 5 cm) is soaked overnight at room temperature in a 1 mM aqueous solution of the elastase chromogenic substrate MeO-Suc-Ala-Ala-Pro-Val-(p-nitroanilide) supplied by Calbiochem Limited. This substrate releases yellow coloured p-nitroanilide dye when the peptide sequence is cleaved by an elastase enzyme. The filter paper is dried at 40°C following the soaking step.

The resulting filter paper has the elastase chromogenic substrate uniformly distributed thereon, and is suitable for mapping elastase concentrations over the surface of a wound.

### Procedure 1

The reactivity of the diagnostic sheet prepared in Example 1 is assessed as follows.

Human mastectomy drain fluid both undiluted and diluted tenfold was dropped onto a sample of the diagnostic wound mapping sheet to test its reactivity towards acute wound fluid.

Human venous ulcer fluid both undiluted and diluted tenfold was likewise dropped onto another sample of the diagnostic wound mapping sheet to determine its reactivity towards chronic wound fluid.

It was found that the acute wound fluid did not develop any colour in the wound mapping sheet. The chronic wound fluid produced an intense yellow colour within 60 seconds, indicative of high elastase activity in the chronic wound fluid.

### Procedure 2

The diagnostic wound mapping sheet prepared in Example 1 is used for diagnostic wound mapping in the following way.

A first (transfer) layer of Whatman No. 1 (RTM) filter paper is used as a blotting layer to transfer wound fluid from a wound surface to a wound mapping sheet prepared as described in Example 1.

The first layer of filter paper is covered with layer a of Tegapore (RTM) microporous polymer film (supplied by 3M Corporation) to prevent cellular debris being transferred from the wound surface. The first layer with Tegapore (RTM) covering is then applied to the surface of a venous ulcer for 30 seconds to blot wound fluid from the wound surface. The first layer is then removed from the wound, separated from the Tegapore covering, and blotted onto the diagnostic wound mapping sheet. After 30 minutes for colour development, the first layer is removed to leave the diagnostic sheet with a coloured wound map thereon.

The wound map shows regions of the wound where the wound fluid contains elevated quantities of elastase. Such areas are likely to exhibit slower healing and ulceration, since the high levels of elastase interfere with the delicate balance of tissue formation and breakdown required for wound healing.

The above embodiments have been described by way of example only.

## Claims

1. A method of providing a map showing the condition of different regions of a wound comprising the steps of:
applying an absorbent sheet to the surface of the wound to blot wound fluid from the wound; followed by
applying the absorbent sheet to a diagnostic sheet to transfer at least a part of said wound fluid to said diagnostic sheet, wherein said diagnostic sheet is selectively reactive over at least a part of its area with one or more molecules present in a wound fluid.

2. A method of providing a map showing the condition of different regions of a wound comprising the step of applying a diagnostic sheet to the surface of the wound, wherein said diagnostic sheet is selectively reactive over at least part of its area with one or more molecules present in a wound fluid.

3. A method according to Claim 1 or 2, wherein the said at least part of its area is greater than 10cm².

4. A method according to any preceding claim, wherein the said diagnostic sheet comprises one or more immunological or other binding partners for the said one or more molecules.

5. A method according to claim 4, wherein said binding partners are immobilised on said diagnostic sheet.

6. A method according to claim 4 or 5, wherein the binding partners have an analog ligand bound thereto, whereby said sheet is suitable for affinity displacement immunoassay.

7. A method according to claim 4, 5 or 6, wherein said binding partners comprise monoclonal antibodies or monoclonal antibody fragments.

8. A method according to any preceding claim, wherein said diagnostic sheet comprises a chemiluminescent, chromogenic or fluorogenic substrate for an enzyme to be mapped in said wound fluid.

9. A method according to claim 8, wherein said enzyme is a protease enzyme, and said substrate comprises a peptide.

10. A method according to any preceding claim, wherein said diagnostic sheet comprises an absorbent layer for absorbing a wound fluid.

11. A method according to any preceding claim, wherein the diagnostic sheet comprises a layer of a material selected from the group consisting of gelatin, cellulose, nitrocellulose, agar, polyacrylamide, starch, alginate, bacterial or plant gums, glass fiber, polyethylene, polycarbonate or polypropylene.

12. A method according to any preceding claim, wherein said one or more molecules with which the diagnostic sheet is reactive are selected from the group consisting of protease enzymes, collagen propeptides, collagen telopeptides, collagen crosslinks such as pyridinoline, protease inhibitors, plasmin, lactate dehydrogenase, cathepsins, cytokines, peroxidase enzymes, cortisol free radicals and growth factors.

13. A method according to claim 12, wherein said one or more molecules are protease enzymes selected from the group consisting of matrix metalloproteinases, low molecular weight gelatinase and elastase.

14. A method according to any of claims 1 to 11, wherein the said one or more molecules comprise an alkaline phosphatase enzyme.

15. A diagnostic sheet for mapping the condition of a wound by application directly to the surface of a wound, wherein said sheet comprises a reactive layer that is selectively reactive over at least a part of its area with one or more molecules present in a wound fluid and a porous wound contacting layer.

16. A diagnostic sheet according to claim 15, wherein the wound contacting layer is microporous.

17. A diagnostic sheet according to claim 15 or 16 for use in a method according to any one of claims 1 to 14.

## Patentansprüche

1. Verfahren zum Bereitstellen einer Aufzeichnung, welche den Zustand von unterschiedlichen Bereichen einer Wunde zeigt, welches die Schritte umfaßt:
Aufbringen eines absorptionsfähigen Blatts auf die Oberfläche der Wunde, um Wundflüssigkeit von der Wunde abzutupfen; gefolgt von
Aufbringen des absorptionsfähigen Blatts auf ein diagnostisches Blatt, um wenigstens einen Teil der Wundflüssigkeit auf das diagnostische Blatt zu transferieren, **dadurch gekennzeichnet, daß** das diagnostische Blatt selektiv reaktiv über wenigstens einen Teil seiner Fläche mit einem oder mehreren Molekülen, die in einer Wundflüssigkeit vorhanden sind, ist.

2. Verfahren zum Bereitstellen einer Aufzeichnung, welche den Zustand von unterschiedlichen Bereichen einer Wunde zeigt, welches den Schritt eines Aufbringens eines diagnostischen Blatts auf die Oberfläche der Wunde umfaßt, **dadurch gekennzeichnet, daß** das diagnostische Blatt selektiv reaktiv über wenigstens einen Teil seiner Fläche mit einem oder mehreren Molekülen, die in einer Wundflüssigkeit vorhanden sind, ist.

3. Verfahren nach Anspruch 1 oder 2, **dadurch gekennzeichnet, daß** der wenigstens eine Teil seiner Fläche größer als 10 cm² ist.

4. Verfahren nach einem der vorangehenden Ansprüche, **dadurch gekennzeichnet, daß** das diagnostische Blatt einen oder mehrere immunologische oder andere Bindungspartner für das eine oder die mehreren Moleküle umfaßt.

5. Verfahren nach Anspruch 4, **dadurch gekennzeichnet, daß** die Bindungspartner auf dem diagnostischen Blatt immobilisiert werden.

6. Verfahren nach Anspruch 4 oder 5, **dadurch gekennzeichnet, daß** die Bindungspartner einen Analogliganden, der daran angebunden ist, aufweisen, wobei das Blatt für ein Affinitätsverschiebungsimmunoassay geeignet ist.

7. Verfahren nach Anspruch 4, 5 oder 6, **dadurch gekennzeichnet, daß** die Bindungspartner monoklonale Antikörper oder monoklonale Antikörperfragmente umfassen.

8. Verfahren nach einem der vorangehenden Ansprüche, **dadurch gekennzeichnet, daß** das diagnostische Blatt ein chemilumineszentes, chromogenes oder fluorogenes Substrat für ein in der Wundflüssigkeit aufzuzeichnendes Enzym umfaßt.

9. Verfahren nach Anspruch 8, **dadurch gekennzeichnet, daß** das Enzym ein Proteaseenzym ist und das Substrat ein Peptid umfaßt.

10. Verfahren nach einem der vorangehenden Ansprüche, **dadurch gekennzeichnet, daß** das diagnostische Blatt eine absorptionsfähige Schicht zum Absorbieren einer Wundflüssigkeit umfaßt.

11. Verfahren nach einem der vorangehenden Ansprüche, **dadurch gekennzeichnet, daß** das diagnostische Blatt eine Schicht aus einem Material umfaßt, das ausgewählt wird aus der Gruppe bestehend aus Gelatine, Cellulose, Nitrocellulose, Agar, Polyacrylamid, Stärke, Alginat, bakteriellen oder pflanzlichen Gummis, Glasfaser, Polyethylen, Polycarbonat oder Polypropylen.

12. Verfahren nach einem der vorangehenden Ansprüche, **dadurch gekennzeichnet, daß** das eine oder die mehreren Moleküle, mit denen das diagnostische Blatt reaktiv ist, ausgewählt werden aus der Gruppe bestehend aus Proteaseenzymen, Collagenpropeptiden, Collagentelopeptiden, Collagenvernetzungen, wie Pyridinolin, Proteaseinhibitoren, Plasmin, Lactatdehydrogenase, Cathepsinen, Cytokinen, Peroxidaseenzymen, Cortisol freien Radikalen und Wachstumsfaktoren.

13. Verfahren nach Anspruch 12, **dadurch gekennzeichnet, daß** das eine oder die mehreren Moleküle Proteaseenzyme sind, die ausgewählt werden aus der Gruppe bestehend aus Matrixmetalloproteinasen, niedermolekulargewichtiger Gelatinase und Elastase.

14. Verfahren nach einem der Ansprüche 1 bis 11, **dadurch gekennzeichnet, daß** das eine oder die mehreren Moleküle ein alkalisches Phosphataseenzym umfassen.

15. Diagnostisches Blatt zum Aufzeichnen des Zustands einer Wunde durch unmittelbares Aufbringen auf die Oberfläche einer Wunde, **dadurch gekennzeichnet, daß** das Blatt eine reaktive Schicht umfaßt, die selektiv reaktiv über wenigstens einen Teil seiner Fläche mit einem oder mehreren Molekülen, die in einer Wundflüssigkeit vorhanden sind, ist, und eine poröse, die Wunde berührende Schicht.

16. Diagnostisches Blatt nach Anspruch 15, **dadurch gekennzeichnet, daß** die die Wunde berührende Schicht mikroporös ist.

17. Diagnostisches Blatt nach Anspruch 15 oder 16 zur Verwendung in einem Verfahren gemäß einem der Ansprüche 1 bis 14.

## Revendications

1. Procédé pour fournir une carte représentant la condition de différentes régions d'une blessure, comportant les étapes consistant à :
appliquer une feuille absorbante sur la surface de la blessure pour éponger un fluide de blessure depuis la blessure, après quoi
appliquer la feuille absorbante sur une feuille de diagnostic pour transférer au moins une partie dudit fluide de blessure sur ladite feuille de diagnostic, ladite feuille de diagnostic étant sélectivement réactive sur au moins une partie de sa surface avec une ou plusieurs molécules présentes dans un fluide de blessure.

2. Procédé pour fournir une carte représentant la condition de différentes régions d'une blessure comportant l'étape consistant à appliquer une feuille de diagnostic sur 1a surface de la blessure, ladite feuille de diagnostic étant sélectivement réactive sur au moins une partie de sa surface avec une ou plusieurs molécules présentes dans un fluide de blessure.

3. Procédé selon la revendication 1 ou 2, dans lequel ladite au moins une partie de sa surface est supérieure à 10 cm².

4. Procédé selon l'une quelconque des revendications précédentes, dans lequel ladite feuille de diagnostic comporte un ou plusieurs partenaires immunologiques ou autres partenaires de liaison pour lesdites une ou plusieurs molécules.

5. Procédé selon la revendication 4, dans lequel lesdits partenaires de liaison sont immobilisés sur ladite feuille de diagnostic.

6. Procédé selon la revendication 4 ou 5, dans lequel les partenaires de liaison ont un ligand analogue lié à ceux-ci, de sorte que ladite feuille est adaptée pour un essai immunologique à déplacement d'affinité.

7. Procédé selon la revendication 4, 5 ou 6, dans lequel lesdits partenaires de liaison comportent des anticorps monoclonaux ou des fragments d'anticorps monoclonaux.

8. Procédé selon l'une quelconque des revendications précédentes, dans lequel ladite feuille de diagnostic comporte un substrat chimioluminescent, chromogénique ou fluorogénique pour une enzyme à localiser dans ledit fluide de blessure.

9. Procédé selon la revendication 8, dans lequel ladite enzyme est une enzyme protéase, et ledit substrat comporte un peptide.

10. Procédé selon l'une quelconque des revendications précédentes, dans lequel ladite feuille de diagnostic comporte une couche absorbante pour absorber un fluide de blessure.

11. Procédé selon l'une quelconque des revendications précédentes, dans lequel la feuille de diagnostic comporta une couche d'un matériau sélectionné parmi le groupe constitué de gélatine, cellulose, nitrocellulose, agar, polyacrylamide, amidon, alginate, gommes bactériennes ou végétales, fibre de verre, polyéthylène, polycarbonate ou polypropylène.

12. Procédé selon l'une quelconque des revendications précédentes, dans lequel lesdites une ou plusieurs molécules avec lesquelles est réactive la feuille de diagnostic sont sélectionnées parmi le groupe constitué d'enzymes protéase, propeptides de collagène, télopeptides de collagène, réticulations de collagène telles qu'une pyridinoline, inhibiteurs de protéase, plasmine, déshydrogénase de lactate, cathepsines, cytokines, enzymes peroxydase, radicaux libres de cortisol et facteurs de croissance.

13. Procédé selon la revendication 12, dans lequel lesdites une ou plusieurs molécules sont des enzymes protéase sélectionnées parmi le groupe constitué de métalloprotéinases de matrice, de gélatinase de faible poids moléculaire et d'élastase.

14. Procédé selon l'une quelconque des revendications 1 à 11, dans lequel lesdites une ou plusieurs molécules comportent une enzyme phosphatase alcaline.

15. Feuille de diagnostic pour cartographier la condition d'une blessure par application directe sur la surface d'une blessure, dans laquelle ladite feuille comporte une couche réactive qui est sélectivement réactive sur au moins une partie de sa surface avec une ou plusieurs molécules présentes dans un fluide de blessure et une couche poreuse de contact de blessure.

16. Feuille de diagnostic selon la revendication 15, dans laquelle la couche de contact de blessure est microporeuse.

17. Feuille de diagnostic selon la revendication 15 ou 16, destinée à être utilisée dans un procédé selon l'une quelconque des revendications 1 à 14.
